# EUROPÄISCHE PATENTSCHRIFT

(11) **EP 2 229 109 B1**
(45) Veröffentlichungstag und Bekanntmachung des Hinweises auf die Patenterteilung: **19.09.2018**
(21) Anmeldenummer: 08853390.6
(22) Anmeldetag: 26.11.2008
(51) Int. Cl.: A61B 17/06, A61L 17/14

(54) **CHIRURGISCHES FADENGEFLECHT**
SURGICAL SUTURE MATERIAL CONSISTING OF BRAIDED THREAD
SOUTURE DE FILS TRESSÉS À USAGE CHIRURGICAL

(30) Priorität: 26.11.2007 DE 102007058256
(43) Veröffentlichungstag der Anmeldung: 22.09.2010
(73) Patentinhaber: Aesculap AG, 78532 Tuttlingen (DE)
(72) Erfinder: BERNDT, Ingo, 52066 Aachen (DE); ODERMATT, Erich, CH-8200 Schaffhausen (CH)
(74) Vertreter: Patentanwälte Ruff, Wilhelm, Beier, Dauster & Partner mbB
(86) Internationale Anmeldenummer: PCT/EP2008/010006
(87) Internationale Veröffentlichungsnummer: WO 2009/068252

(56) Entgegenhaltungen:
- EP-A- 0 916 312
- WO-A-2006/026397
- WO-A-2007/120138
- US-A1- 2005 149 118
- US-A1- 2007 027 475

## Beschreibung

Die vorliegende Erfindung betrifft ein chirurgisches Nahtmaterial in Form eines Fadengeflechts, ein Verfahren zu seiner Herstellung, ein Kit sowie Verwendungen des Nahtmaterials.

Zum Verschluss von Wunden werden in der Chirurgie standardmäßig fadenförmige Nahtmaterialien eingesetzt. Diese werden gewöhnlich geknotet, um eine sichere Verankerung mit den zu verschließenden Geweben zu gewährleisten. Das Knoten von chirurgischem Nahtmaterial hat hierbei einen maßgeblichen Einfluss auf die Qualität des Wundverschlusses, da über die Qualität nicht nur die physikalisch-chemischen Eigenschaften von Nahtmaterialien, sondern auch eine korrekte Knotentechnik durch den Chirurgen entscheiden.

Das Erlernen der korrekten Knotentechnik ist eine anspruchsvolle und vor allem langwierige Übung. Dem Erlernen dieser Technik wird in der heutigen Chirurgenausbildung jedoch häufig nicht in ausreichendem Maß Zeit und Beachtung geschenkt. Daher stellt das Knoten von chirurgischem Nahtmaterial eine häufige Ursache für das Auftreten von sogenannten Wunddehiszenzen dar. Hauptfehlerquellen sind hierbei vor allem eine falsche Knotenwurfrichtung, eine falsche Knotenwahl, zu wenig oder zu fest angezogene Knoten sowie eine dem Wundmilieu nicht angepasste Positionierung der einzelnen Würfe. Zudem müssen oftmals mehrere Knoten, insbesondere bis zu 7 Knoten, für einen sicheren Knotenhalt übereinandergesetzt werden. Dies stellt einen hohen Materialeintrag in das Gewebe dar und kann zu verstärkten Fremdkörperreaktionen führen.

Daher sind schon seit längerer Zeit knotenlose bzw. selbstfixierende Nahtmaterialien in den Fokus der Nahtmaterialforschung gelangt. Bislang bekannt sind sogenannte "barbed sutures", auch "self-locking sutures" oder "self-retaining sutures" genannt. Diese bestehen gewöhnlich aus einem monofilen Faden, der entlang seiner Längsachse widerhakenförmige Strukturen, so genannte "barbs", aufweist. Diese werden gewöhnlich durch Einschnitte in das Fadenmaterial erzeugt. Dadurch können die Fäden entlang der Richtung der Widerhaken durch das Gewebe gezogen werden. Bei Zug in die entgegengesetzte Richtung stellen sich die Widerhaken auf und verankern sich und damit das Nahtmaterial im Gewebe, indem jeder Widerhaken seinen eigenen kleinen, schräg verlaufenden Stichkanal bohrt. Auf diese Weise wird verhindert, dass das Nahtmaterial durch den Stichkanal zurückgezogen werden kann. Diese "barbed sutures" sind aus dem Stand der Technik hinlänglich bekannt. Beispielsweise geht ein derartiges Nahtmaterial aus der WO 2004/030520 A2 hervor. Nachteilig ist jedoch die Monofilamentstruktur, die diesen Nahtmaterialien normalerweise zugrunde liegt. Monofilamente sind allgemein eher steife Strukturen und daher unhandlich in der Handhabung. Außerdem stellen die in das Monofilament eingeschnittenen Widerhaken Schwachstellen im Nahtmaterial dar, die dessen mechanische Belastbarkeit reduzieren. So ist aus der Fachliteratur unter anderem bekannt, dass die "barbs" zu einer Verringerung der Linearreißkraft LTS (Linear Tensile Strength, LTS) um eine USP Stärke (Stärke nach United States Pharmacopeia) führen können (R. Rashid, Arch. Dermatol. 2007, 143 (7), 869-872).

Ein Beispiel für ein geflochtenes Nahtmaterial mit Widerhaken, welche aus einem Fadengeflecht herausragen, lässt sich der US 2007/0005110 A2 entnehmen. Bei diesem Nahtmaterial sind Filamente ohne Widerhaken und mindestens ein Filament mit Widerhaken derart miteinander verflochten, dass die Filamente ohne Widerhaken das Filament mit den Widerhaken ummanteln, wobei die Widerhaken aus der Ummantelung herausragen. Unberührt hiervon bleibt jedoch eine gewisse Schwächung des die Widerhaken aufweisenden, umflochtenen Filaments.

Ein gewebtes Nahtmaterial mit abstehenden Fadengebilden ist aus der WO 2006/026397 A2 bekannt.

Die WO 2007/120138 A2 offenbart ein Wundverschlussgitter mit perforierten tubulären Gitterbestandteilen.

Aus der US 2007/0027475 A1 ist ein Nahtmaterial mit einer texturierten Oberfläche bekannt.

Gegenstand der EP 0 916 312 A1 ist ein Nahtmaterialanker mit einem schraubenförmigen Gewinde.

Die US 2005/0149118 A1 offenbart ein Nahtmaterial mit einem Kern-Mantel-Aufbau, wobei der Kern bioabsorbierbare Fäden und der Mantel eine Flechtstruktur mit nicht absorbierbaren Fäden sowie bioabsorbierbaren Fäden aufweist.

Die vorliegende Erfindung stellt sich daher die Aufgabe, ein chirurgisches Nahtmaterial bereitzustellen, das einen knotenlosen Verschluss und/oder eine knotenlose Fixierung von biologischen Geweben erlaubt und insbesondere aus dem Stand der Technik bekannte Nachteile vermeidet.

Diese Aufgabe wird erfindungsgemäß gelöst durch ein chirurgisches Nahtmaterial in Form eines Fadengeflechts, wobei das Nahtmaterial zur Verankerung in biologischen Geweben aus dem Fadengeflecht herausragende Fadengebilde aufweist.

Durch die Erfindung wird ein chirurgisches multifiles Nahtmaterial bereitgestellt, dessen herausragende (abstehende) Fadengebilde eine knotenlose Fixierung oder Verankerung des Nahtmaterials in einem biologischen Gewebe ermöglichen. Die Fadengebilde werden nach der Implantation bei Zugbelastung des Nahtmaterials in der der Durchzugsrichtung entgegengesetzten Richtung aufgestellt und verankern dadurch das Nahtmaterial im Gewebe, indem sie sich in das dem Hauptstichkanal benachbarte Gewebe bohren. Die abstehenden Fadengebilde fungieren hierbei in vorteilhafter Weise als Fixierungs- oder Verankerungsstrukturen. Eine Materialschwächung des Nahtmaterials, wie sie vor allem bei der Herstellung von "barbs" verursacht wird, kann auf diese Weise vermieden werden, da in der Regel genügend durchgehende Fadenabschnitte anderer Fäden vorhanden sind, die die Zugkräfte aufnehmen. Außerdem kann die Richtung der herausragenden Fadengebilde noch nachträglich eingestellt und damit individuell angepasst werden.

Bei den Fadengebilden handelt es sich um Fadenschlingen. Bei den Fadenschlingen kann es sich um Überfütterungen, Flottungen und/oder Veloursschlingen handeln. Fadenschlingen besitzen den Vorteil, dass sie beim Einziehen des erfindungsgemäßen Nahtmaterials in ein Gewebe weniger Widerstand bieten und damit das Risiko einer Gewebetraumatisierung verringern.

Alternativ handelt es sich bei den herausragenden Fadengebilden um geöffnete Fadenschlingen, insbesondere geöffnete Flottungen, geöffnete Überfütterungen und/oder geöffnete Veloursschlingen. Beispielsweise kann es sich bei den herausragenden Fadengebilden um durchgeschnittene Fadenschlingen handeln.

Die herausragenden Fadengebilde liegen vorzugsweise verstärkt, insbesondere versteift, vor. Die Fadengebilde können physikalisch, beispielsweise mechanisch, oder chemisch verstärkt sein. So können die Fadengebilde insbesondere durch Polymere verstärkt sein. Hierzu können die Fadengebilde beispielsweise in flüssige Polymere oder in Polymerlösungen getaucht werden, wobei die Polymere anschließend ausgehärtet werden. Weiterhin können die Fadengebilde thermisch fixiert sein. Die Fadengebilde können auch verschweißt, vorzugsweise ultraverschweißt, sein.

Die Fadengebilde können grundsätzlich in unterschiedlichen Anordnungen auf dem Fadengeflecht ausgebildet sein. So können die Fadengebilde in einer reihenförmigen Anordnung, versetzten Anordnung, zickzackförmigen Anordnung, spiralförmigen Anordnung, statistischen Anordnung oder in Kombinationen davon auf dem Fadengeflecht angeordnet sein. Bevorzugt sind die herausragenden Fadengebilde in einer regelmäßigen Verteilung auf dem Fadengeflecht angeordnet. Beispielsweise können die herausragenden Fadengebilde hintereinander in Form mindestens einer Reihe, insbesondere einer, zwei, drei oder mehrerer Reihen, vorzugsweise in Längsrichtung des Fadengeflechts angeordnet sein.

Weiterhin kann das Fadengeflecht auf seiner Oberfläche Flächenbereiche aufweisen, die frei von aus der Flechtstruktur herausragenden Fadengebilden sind. Insbesondere können sich Flächenbereiche, die Fadengebilde aufweisen, mit Flächenbereichen, die keine Fadengebilde aufweisen, auf der Oberfläche der Flechtstruktur abwechseln. Erfindungsgemäß kann es daher vorgesehen sein, dass auf der Oberfläche der Flechtstruktur Flächenbereiche mit herausragenden Fadengebilden voneinander beabstandet sind.

In einer weiteren Ausführungsform besitzen die herausragenden Fadengebilde auf dem Fadengeflecht eine sogenannte bidirektionale Anordnung. Unter einer bidirektionalen Anordnung soll hierbei eine Anordnung verstanden werden, bei welcher die Fadengebilde in zwei unterschiedliche Richtungen orientiert sind. Bevorzugt sind die Fadengebilde, vorzugsweise in Längsrichtung des Fadengeflechts betrachtet, für einen ersten Fadengeflechtsabschnitt in Richtung eines übrigen zweiten Fadengeflechtsabschnitts und für den übrigen zweiten Fadengeflechtsabschnitt in Richtung des ersten Fadengeflechtsabschnitts ausgebildet. Besonders bevorzugt sind die Fadengebilde, vorzugsweise in Längsrichtung des Fadengeflechts betrachtet, für einen ersten Fadengeflechtsabschnitt in Richtung Mitte des Fadengeflechts und für einen übrigen zweiten Fadengeflechtsabschnitt ebenso in Richtung Mitte des Fadengeflechts orientiert. Bevorzugt entspricht die Länge der Fadengeflechtsabschnitte in etwa der Hälfte des Fadengeflechts. Bei den Fadengeflechtsabschnitten kann es sich um Umfangs-, Flächen- oder Längenabschnitte des Fadengeflechts handeln.

Die herausragenden Fadengebilde besitzen zweckmäßigerweise einen gewissen Mindestabstand zueinander. Insbesondere halten Fadengebilde, die vorzugsweise aus einem einzigen Faden gebildet werden, einen bestimmten Mindestabstand zueinander. Mit besonderem Vorteil wird der Faden dadurch ausreichend fest, beispielsweise durch Reibung und/oder Bekneifung, im Fadengeflecht gehalten und kann damit nicht ohne Weiteres aus dem Fadengeflecht herausgezogen werden. Bevorzugt weisen die herausragenden Fadengebilde, vorzugsweise in Längsrichtung des Nahtmaterials, einen Abstand von 0,2 bis 10 mm, insbesondere 0,5 bis 5 mm, vorzugsweise 0,5 bis 3 mm, zueinander auf, gemessen von den Austrittsstellen der Fadengebilde aus dem Fadengeflecht. Fadengebilde, die aus einem einzigen Faden gebildet sind, können einen größeren Abstand voneinander haben.

Das Fadengeflecht kann aus mono- und/oder multifilen Fäden (Mono- und/oder Multifilamenten) gebildet sein. Eine Kombination aus monofilen und multifilen Fäden ist bevorzugt, da durch die Verwendung von multifilen Fäden das Fadengeflecht insgesamt geschmeidiger und insbesondere flexibler wird im Vergleich zu einer Flechtstruktur aus monofilen Fäden. Des Weiteren weist ein Fadengeflecht mit multifilen Fäden in der Regel einen verringerten oder keinen "Memory-Effekt" auf.

In einer bevorzugten Ausführungsform weisen die Fäden mit den herausragenden Fadengebilden einen Anteil von 2 bis 80%, bevorzugt 2 bis 50%, insbesondere 5 bis 35%, insbesondere 10 bis 15%, bezogen auf die Gesamtzahl an Fäden im Fadengeflecht, auf. Die Fadengebilde schließen mit der Oberfläche des Fadengeflechts vorzugsweise einen Winkel α < 90°, insbesondere zwischen 5 und 70°, ein.

Das Fadengeflecht weist in einer weiteren Ausführungsform Mehrfachfäden (beim Flechtvorgang mehrfach genommene Fäden), insbesondere Doppel- und/oder Tripelfäden, auf. Die Mehrfachfäden weisen typischerweise Mono- und/oder Multifilamente auf. Erfindungsgemäß können die Mehrfachfäden selbst Flechtstrukturen, insbesondere geflochtene Multifilamente, aufweisen. Bevorzugt weisen die Mehrfachfäden zumindest ein Monofilament auf. Die herausragenden Fadengebilde können grundsätzlich von Mehrfachfäden, insbesondere Doppel- und/oder Tripelfäden, stammen. Bei den herausragenden Fadengebilden handelt es sich um Einzelfäden, insbesondere monofile Einzelfäden, die von Mehrfachfäden, insbesondere Doppel- und/oder Tripelfäden, stammen. Das Fadengeflecht selbst kann beispielsweise aus zwei Einfachfäden und einem Doppelfaden und/oder aus drei Doppelfäden und zwei Einzelfäden gebildet sein. In der Regel weist das erfindungsgemäße Geflecht jedoch deutlich mehr Fäden, insbesondere Einzel- und/oder Mehrfachfäden, auf. Die Verwendung von Mehrfachfäden hat den Vorteil, dass sich zumindest ein Einzelfaden des Mehrfachfadens durchgehend am Aufbau der Flechtstruktur beteiligen kann, während die restlichen Einzelfäden des Mehrfachfadens an gleichen oder verschiedenen Stellen aus der Flechtstruktur herausgeführt werden können. Dies wirkt sich auch vorteilhaft auf die textilen Eigenschaften, wie beispielsweise die lineare Reißkraft, des erfindungsgemäßen Nahtmaterials aus.

Vorzugsweise stammen die aus der Flechtstruktur herausragenden Fadengebilde von steiferen, insbesondere biegesteifen, dickeren oder sperrigeren Fäden als die übrigen Fäden des Fadengeflechts. Eine höhere Biegesteifigkeit kann insbesondere durch eine monofile Struktur der herausragenden Fadengebilde bedingt sein. Deswegen ist es erfindungsgemäß bevorzugt, wenn die herausragenden Fadengebilde von monofilen Fäden des Fadengeflechts stammen. Die herausragenden Fadengebilde stammen bevorzugt von monofilen Fäden des Fadengeflechts. Monofilamente weisen grundsätzlich eine höhere Steifigkeit als Multifilamente auf. Erfindungsgemäß kann es daher vorgesehen sein, die steifen Eigenschaften von Monofilamenten für eine sichere Verankerung des erfindungsgemäßen Nahtmaterials in einem biologischen Gewebe auszunutzen. Weiterhin kann es vorgesehen sein, dass die Fäden mit den herausragenden Fadengebilden Monofilamente und die übrigen Fäden des Fadengeflechts Multifilamente sind. Weiterhin lässt sich eine höhere Steifigkeit, insbesondere Biegesteifigkeit, der herausragenden Fadengebilde auch durch einen größeren Durchmesser, eine höhere intrinsische Biegesteifigkeit (Biegemodul) und/oder eine höhere Härte des Fadenmaterials erzielen, aus dem die Fadengebilde hergestellt sind.

Erfindungsgemäß kann es weiterhin bevorzugt sein, dass das Fadengeflecht Fäden von unterschiedlichem Titer aufweist. Vorzugsweise weisen die Fäden, deren Gebilde aus dem Fadengeflecht herausragen, einen höheren Titer als die restlichen (übrigen) Fäden des Fadengeflechts auf. Unter den restlichen bzw. übrigen Fäden sollen hierbei die Fäden verstanden werden, welche sich durchgehend am Aufbau der Flechtstruktur beteiligen. Ein höherer Titer bedeutet in diesem Zusammenhang ein größeres Verhältnis von Fadenmasse zu Fadenlänge. Bei gleicher Dichte der Fäden bedeutet dies weiterhin ein höheres Verhältnis von Fadendurchmesser zu Fadenlänge. Bevorzugt stammen die herausragenden Fadengebilde von Fäden mit einem Titer zwischen 10 und 2500 dtex, insbesondere 10 und 1700 dtex, bevorzugt 20 und 700 dtex.

Erfindungsgemäß kann es weiterhin vorgesehen sein, dass das Fadengeflecht Fäden von unterschiedlicher Biegesteifigkeit aufweist. Bevorzugt weisen die Fäden, deren Gebilde aus dem Fadengeflecht herausragen, eine höhere Biegesteifigkeit als die restlichen (übrigen) Fäden des Fadengeflechts auf. Die höhere Biegesteifigkeit kann zum einen auf einem höheren Titer der herausragenden Fadengebilde beruhen. Alternativ oder in Kombination dazu lässt sich die Biegesteifigkeit der herausragenden Fadengebilde aber auch durch eine nachträgliche Verstärkung der Fadengebilde, insbesondere durch Verschweißen oder durch eine chemische oder physikalische Behandlung, erhöhen. Dadurch kann mit besonderem Vorteil die Fixierung des erfindungsgemäßen Nahtmaterials in einem biologischen Gewebe zusätzlich verbessert werden. Die herausragenden Fadengebilde stammen vorzugsweise von Fäden mit einer Biegesteifigkeit zwischen 10 und 600 mN, insbesondere 5 und 560 mN.

Bevorzugt besitzen die herausragenden Fadengebilde eine Länge zwischen 0,05 und 3 mm, insbesondere 0,05 mm und 2 mm, vorzugsweise 0,20 und 1,5 mm. Insbesondere können die Fäden, deren Gebilde aus dem Fadengeflecht herausragen, einen Durchmesser zwischen 30 und 250 µm, insbesondere 70 und 150 µm, aufweisen. Die Fadengebilde können dabei einen runden, ovalen, dreieckigen, quadratischen, trapezoidalen, rhomboiden, pentagonalen bzw. fünfeckigen, hexagonalen bzw. sechseckigen, stern- oder kreuzförmigen Querschnitt aufweisen.

Grundsätzlich kommen zur Herstellung des Nahtmaterials alle bioverträglichen Materialien in Betracht. Bei den Materialien kann es sich um Polymere, insbesondere um Co- und/oder Terpolymere, handeln. Die Materialien können zudem als Blockpolymere, insbesondere Blockcopolymere und/oder Blockterpolymere, vorliegen.

In einer möglichen Ausführungsform ist das Nahtmaterial aus nicht resorbierbaren Polymeren, insbesondere aus Polyurethanen, Polyestern, Polyamiden, Polyolefinen, Copolymeren davon, Terpolymeren davon und/oder Mischungen davon, gebildet. Als geeigneter Polyester kommt vor allem Polyethylenterephthalat in Betracht. Ein Beispiel für ein mögliches Polyolefin ist Polypropylen. Die in Frage kommenden Polyolefine können weiterhin halogeniert sein. Beispielsweise kann es sich bei den Polyolefinen auch um Polyvinylidendifluorid (PVDF) und/oder Polytetrafluorethylen, insbesondere expandiertes Polytetrafluorethylen, handeln. Beispiele für mögliche Polyamide sind Polyamid 6.6 oder Polyamid 6.

Weiterhin kann das Nahtmaterial erfindungsgemäß aus resorbierbaren Polymeren gebildet sein. Beispiele für geeignete resorbierbare Polymere sind insbesondere Polyglykolid, Polylactid, Poly-E-caprolacton, Polytrimethylencarbonat, Poly-p-dioxanon, 4-Polyhydroxybuttersäure und/oder Mischungen davon. Des Weiteren kann es sich im Falle von resorbierbaren Polymeren um Co- oder Terpolymere, insbesondere Blockco- und/oder Blockterpolymere, umfassend zumindest ein Monomer aus der Gruppe Glykolid, Lactid, ε-Caprolacton, Trimethylencarbonat, Para-Dioxanon und 4-Polyhydroxybuttersäure, handeln.

Als geeignete Materialien zur Herstellung des erfindungsgemäßen Nahtmaterials kommen insbesondere die von der Anmelderin unter den Bezeichnungen Monosyn®, MonoPlus®, Dafilon®, Premilene®, und/oder MonoMax® kommerziell vertriebenen Nahtmaterialien in Frage. Monosyn® ist ein synthetisches monofiles Nahtmaterial aus Polyglykolid oder einem Copolymer aus Glykolid und Lactid. Dafilon® ist ein nicht resorbierbares monofiles Nahtmaterial aus Polyamid 6 oder Polyamid 6.6. Premilene® ist ein nicht resorbierbares monofiles Nahtmaterial aus Polypropylen. MonoPlus® ist ein langzeitresorbierbares monofiles Nahtmaterial aus Polydioxanon. MonoMax® ist ein langfristig resorbierbares Monofilament aus 4-Polyhyroxybuttersäure.

Als Fadenstärken für das erfindungsgemäße Nahtmaterial kommen die üblicherweise verwendeten Fadenstärken in Betracht, insbesondere mindestens eine Fadenstärke aus der Gruppe USP 8/0, USP 7/0, USP 6/0, USP 5/0, USP 4/0, USP 3/0, USP 2/0, USP 0, USP 1, USP 2, USP 3, USP 4, USP 5 und USP 6.

In einer weiteren Ausführungsform ist das Nahtmaterial aus resorbierbaren und nicht resorbierbaren Materialien gebildet. So kann das Nahtmaterial sowohl resorbierbare als auch nicht resorbierbare Fäden aufweisen. Im Falle von Mehrfachfäden können diese aus resorbierbaren und nicht resorbierbaren Einzelfäden bestehen. In einer weitergehenden Ausführungsform sind die herausragenden Fadengebilde aus einem resorbierbaren und einem nicht resorbierbaren Material gebildet, wobei vorzugsweise das resorbierbare Material das nicht resorbierbare Material ummantelt oder umgekehrt (Fäden mit Kern-Mantel-Aufbau, insbesondere Bikomponentenfäden). Bezüglich der in Frage kommenden Materialien wird auf die bisherige Beschreibung Bezug genommen.

Das Fadengeflecht kann grundsätzlich bioaktive Substanzen aufweisen. Bevorzugt weist das Fadengeflecht Wachstumsfaktoren, entzündungshemmende Verbindungen, schmerzstillende Substanzen und/oder antimikrobielle Wirkstoffe auf. Bei den antimikrobiellen Wirkstoffen kann es sich um antimikrobielle, insbesondere antibakterielle, Zusammensetzungen oder Verbindungen handeln. Beispielsweise kann es sich bei den Wirkstoffen um antimikrobielle Metalle, Metalllegierungen oder Metallsalze handeln. Als antimikrobielle Metalle kommen grundsätzlich Silber, Kupfer, Zink und/oder Gold in Betracht. Bevorzugt ist der antimikrobielle Wirkstoff Silber oder ein Silbersalz. Die antimikrobiellen Metalle bzw. deren Salze, beispielsweise Oxide, können in Form von Nanopartikeln und/oder Mikropartikeln vorliegen. Als weitere antimikrobielle Wirkstoffe kommen beispielsweise Triclosan, Chlorhexidin und/oder Polyhexamethylenbiguanid in Frage.

In einer weiteren Ausführungsform ist das Fadengeflecht ein Flachgeflecht. In dieser Ausführungsform ist das Fadengeflecht normalerweise aus einer ungeraden Anzahl von Fäden gebildet. Das Fadengeflecht kann insbesondere bandförmig vorliegen. In einer weitergehenden Ausführungsform ist das Fadengeflecht ein Flachgeflecht, wobei die herausragenden Fadengebilde nur auf einer Seite, insbesondere einer Flächenseite, des Flachgeflechts angeordnet sind. Erfindungsgemäß ist es zudem möglich, dass die herausragenden Fadengebilde auf beiden Seiten, insbesondere beiden Flächenseiten, eines Flachgeflechts angeordnet sind. Weiterhin kann das Fadengeflecht ein Flachgeflecht sein, wobei die herausragenden Fadengebilde nur an den Rändern des Flachgeflechts angeordnet sind. Auch Kombinationen sind möglich. Bezüglich weiterer Merkmale und Einzelheiten, insbesondere im Hinblick auf mögliche Anordnungen und Orientierungen der Fadengebilde auf dem Fadengeflecht, wird vollständig auf die bisherige Beschreibung Bezug genommen.

In einer bevorzugten Ausführungsform ist das Fadengeflecht ein Rund- oder Schlauchgeflecht. In dieser Ausführungsform ist das Fadengeflecht üblicherweise aus einer geraden Anzahl von Fäden gebildet. Das Rund- bzw. Schlauchgeflecht weist vorzugsweise eine Seele auf. Die Seele selbst kann aus einem resorbierbaren Material bestehen. Bezüglich weiterer Eigenschaften der Seele, insbesondere hinsichtlich des Materials aus dem die Seele herstellbar ist, wird auf die bisherige Beschreibung Bezug genommen. Ebenso wird auf die bisherige Beschreibung verwiesen, was beispielsweise mögliche Anordnungen und Orientierungen der Fadengebilde auf dem Fadengeflecht anbelangt.

Die vorliegende Erfindung betrifft weiterhin ein Verfahren zur Herstellung des Nahtmaterials, wobei zur Herstellung des Fadengeflechts Fäden unter Ausbildung einer Flechtstruktur miteinander geflochten werden und einzelne Fäden zur Ausbildung von aus der Flechtstruktur herausragenden Fadengebilden während des Flechtens aus der sich bildenden Flechtstruktur herausgeführt werden. Wie bereits erwähnt, kann es sich bei den einzelnen Fäden um Fäden der Flechtstruktur, beispielsweise um Einzelfäden von Mehrfachfäden, und/oder um Fäden einer Seele der Flechtstruktur handeln.

Mit Vorteil wird die Fadenzahl der Einzelfäden über die Länge des Nahtmaterials im Wesentlichen konstant gehalten. Bevorzugt werden die herausgeführten Fäden wieder in die Flechtstruktur und/oder andere Fäden in die Flechtstruktur eingeführt, um die Fadenzahl im Wesentlichen konstant zu halten. Erfindungsgemäß ist es insbesondere vorgesehen, dass nach jedem Herausführen eines Fadens ein Faden, insbesondere ein neuer Faden und/oder der bisherige Faden, in die Flechtstruktur eingefügt wird. Auf diese Weise kann eine zunehmende Verjüngung der Flechtstruktur vermieden werden.

Die herausgeführten Fäden werden unter Ausbildung von Schlingen, insbesondere nach Art von Flottungen, Überfütterungen und/oder Veloursschlingen, wieder in die Flechtstruktur hineingeführt. In dieser Ausführungsform können die ausgebildeten Fadenschlingen als Verankerungsstrukturen des Nahtmaterials dienen. Gemäß einer weitergehenden Ausführungsform werden die ausgebildeten Fadenschlingen unter Ausbildung der herausragenden Fadengebilde geöffnet, vorzugsweise durchgeschnitten. Die in diesem Abschnitt beschriebenen Ausführungsformen besitzen den Vorteil, dass eine kontinuierliche Flechtung zur Herstellung des erfindungsgemäßen Nahtmaterials möglich ist. Die Schlingen können durch Überfütterung des jeweiligen Fadens begünstigt werden. Bei Mehrfachfäden kann dann mindestens ein Einzelfaden überfüttert sein.

Das Öffnen der Fadenschlingen kann grundsätzlich mittig oder an einer davon abweichenden Position vorgenommen werden. Entsprechend können pro Schlinge zwei aus dem Fadengeflecht herausragende Fadengebilde erzeugt werden, welche entweder mit gleicher Länge oder mit verschiedenen Längen aus dem Fadengeflecht herausragen. Erfindungsgemäß kann es insbesondere vorgesehen sein, dass jeweils eines dieser Fadengebilde möglichst dicht über der Flechtstruktur abgeschnitten wird. Das andere Fadengebilde weist dann eine Orientierung in eine Richtung auf. Auf diese Weise können Fadengeflechte hergestellt werden, deren herausragende Fadengebilde in verschiedene Richtungen zeigen.

In einer weiteren Ausführungsform werden die herausragenden Fadengebilde verstärkt, insbesondere versteift. Die Verstärkung der Fadengebilde kann dabei nachträglich, dass heißt nach Herstellung der Flechtstruktur, vorgenommen werden. Die Fadengebilde können beispielsweise verschweißt, insbesondere ultraverschweißt, werden. Eine chemische Verstärkung, insbesondere Verfestigung, kann ebenfalls durchgeführt werden und kann beispielsweise mittels einer Beschichtung mit Polymeren vorgenommen werden. Durch die in diesem Abschnitt beschriebenen Maßnahmen kann die Biegesteifigkeit der Fadengebilde mit besonderem Vorteil erhöht werden.

Weiterhin können die Fadengebilde einer nachträglichen Orientierung, d.h. nach Herstellung der Flechtstruktur, unterworfen werden. Hierbei kann es sich um eine rein mechanische Orientierung oder um eine thermische, d.h. in Folge von Hitzeeinwirkung bedingte, Orientierung handeln.

In einer bevorzugten Ausführungsform werden die Fäden beim Flechten mindestens doppelt genommen. Auf diese Weise kann eine Beeinträchtigung der Flechtstruktur, insbesondere beim Öffnen von in den vorherigen Abschnitten beschriebenen Fadenschlingen, weitgehend vermieden werden.

In einer weitergehenden Ausführungsform wird zumindest ein Ende des erfindungsgemäßen Nahtmaterials mit einer chirurgischen Nadel armiert. Zur Armierung mit einer chirurgischen Nadel wird das Nahtmaterial in aller Regel in eine hierfür vorgesehene Nadelbohrung gesteckt und die Nadel anschließend im Bereich der Bohrung zusammengepresst.

Ein weiterer Aspekt der vorliegenden Erfindung betrifft ein chirurgisches Kit bzw. Set, umfassend das erfindungsgemäße Nahtmaterial und zumindest eine chirurgische Nadel. Bezüglich weiterer Merkmale und Einzelheiten zu dem Kit bzw. Set wird auf die bisherige Beschreibung Bezug genommen.

Die Erfindung betrifft außerdem die Verwendung des Nahtmaterials als selbstfixierendes oder knotenloses Nahtmaterial. Das Nahtmaterial eignet sich vor allem für Indikationen, bei denen das kosmetische Ergebnis für den Patienten von besonderer Bedeutung ist. Daher betrifft ein weiterer Aspekt der vorliegenden Erfindung die Verwendung des Nahtmaterials in der plastischen Chirurgie und/oder Wiederherstellungschirurgie, insbesondere zum Hautverschluss. Auf dem Gebiet der plastischen Chirurgie eignet sich das Nahtmaterial beispielsweise zum Face-Lift und/oder zur Augenbrauenhebung. Das Nahtmaterial kann insbesondere zum intrakutanen, subkutanen oder oberflächlichen Hautverschluss verwendet werden.

Weiterhin eignet sich das erfindungsgemäße Nahtmaterial für Wundbereiche, die eine Knotung von herkömmlichen Nahtmaterialien erschweren oder sogar unmöglich machen. Beispielsweise kann das erfindungsgemäße Nahtmaterial in der abdominalen, gynäkologischen und/oder urologischen Chirurgie verwendet werden. Weitere Anwendungsgebiete betreffen die Mikro-, Augen-, Neuro-, Gefäß-, Herz-, Magen- und Darmchirurgie. Das Nahtmaterial eignet sich weiterhin zur Verwendung in der endoskopischen und/oder laparoskopischen Chirurgie. Das Nahtmaterial eignet sich außerdem zum Verschluss von Trokarinzisionen, insbesondere mit sogenannten alpha-Stichen. Das Nahtmaterial eignet sich ferner auch zum Verschluss von inneren Wunden.

Ein weiterer Aspekt der Erfindung betrifft schließlich die Verwendung des Nahtmaterials zur Fixierung von Implantaten, insbesondere von Herniennetzen, vorzugsweise von Herniennetzen im peritonealen Körperbereich. Darüber hinaus lassen sich grundsätzlich auch andere Implantate, beispielsweise Prolapsnetze und/oder Harninkontinenznetze, mittels des erfindungsgemäßen Nahtmaterials fixieren.

Weitere Merkmale und Einzelheiten der Erfindung ergeben sich aus den nachfolgenden Beispielen und Figuren in Kombination mit den Unteransprüchen. Sämtliche Figuren werden hiermit durch ausdrückliche Bezugnahme zum Inhalt dieser Beschreibung gemacht. Dabei können einzelne Merkmale jeweils für sich alleine oder zu mehreren in Kombination miteinander verwirklicht sein.

In den Figuren ist schematisch gezeigt:
- Figur 1 und 2:: Ausführungsformen eines erfindungsgemäßen Nahtmaterials mit verschiedenen abstehenden Fadengebilden,
- Figur 3 und 4:: Ausführungsform eines erfindungsgemäßen Nahtmaterials mit Tripelfäden,
- Figur 5:: Ausführungsform eines erfindungsgemäßen Nahtmaterials mit entgegengesetzt orientierten, abstehenden Fadenenden,

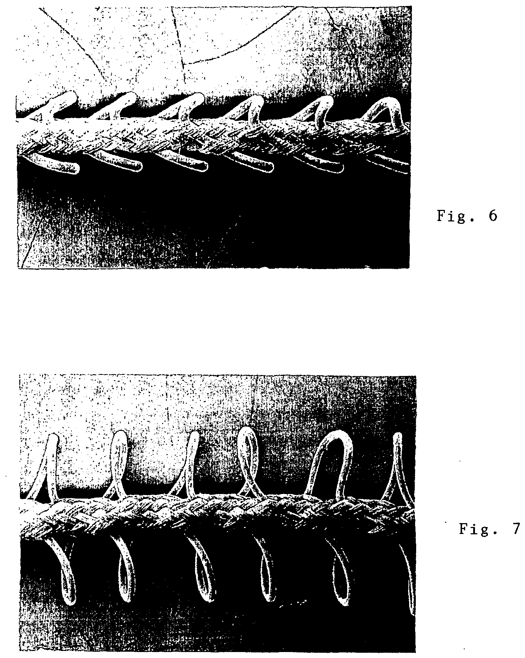

- Figur 6:: REM-Aufnahme eines erfindungsgemäßen Nahtmaterials,
- Figur 7:: REM-Aufnahme eines erfindungsgemäßen Nahtmaterials.

### Beispiele

### Beispiel 1:

Zwei einzelne monofile Fäden aus Medical Grade Polypropylen und ein Doppelfaden aus Medical Grade Polypropylen der Stärke USP 5/0 werden nach Art einer Strangflechtung miteinander verflochten. Alle zehn Flechten wird ein Faden des Doppelfadens aus dem Geflecht herausgeführt und überfüttert wieder in das Geflecht hineingeführt. Die aus dem Geflecht herausstehenden Schlingen (Schlaufen) werden anschließend unter dem Mikroskop mit einer Schneideeinrichtung mit einer Klinge bei einer Länge von ca. 5/6 der Schlaufenlänge geöffnet. Anschließend wird das kurze Ende der geöffneten Schlaufe thermisch fixiert.

### Beispiel 2:

Drei Doppelfäden aus Medical Grade Polypropylenmonofilamenten der Stärke USP 6/0 werden nach Art einer Strangflechtung miteinander verflochten. Nach jeder sechsten Flechte wird ein Polypropylenmonofilament aus dem Geflecht herausgeführt, überfüttert und wieder in das Geflecht eingeführt. Die Schlingen bzw. Schlaufen werden wie in Beispiel 1 geöffnet. Die kurzen Enden der geöffneten Schlaufen werden anschließend thermisch fixiert. Die herausgeführten Einzelfäden stammen dabei immer von Doppelfäden, die der Reihe nach wechseln. Auf diese Weise stammt nur jedes dritte herausgeführte Fadenende von ein und demselben Doppelfaden ab (Abstand: 18 Flechten).

### Beispiel 3:

Ein Tripelfaden aus Medical Grade Polypropylenmonofilamenten und zwei Einzelfäden der Stärke USP 6/0 werden nach Art einer Strangflechtung miteinander verflochten. Alle zehn Flechten wird zunächst der erste Einzelfaden des Tripelfadens aus dem Fadengeflecht herausgeführt und entsprechend Beispiel 1 geöffnet und fixiert. Nach einer weiteren Flechte wird der zweite Einzelfaden des Tripelfadens aus dem Geflecht herausgeführt und fixiert. Auf diese Weise treten die herausgeführten Fadenenden paarweise entlang der Länge des Fadens auf.

### Beispiel 4:

Drei Doppelfäden bestehend aus einem Medical Grade Polypropylenmonofilament der Stärke USP 6/0 und einem geflochtenen Multifilament aus Medical Grade Polyethylenterephthalat werden nach Art einer Strangflechtung miteinander verflochten. Wie in Beispiel 2 wird jeweils das Monofilament alle sechs Flechten aus dem Geflecht herausgeführt, überfüttert und fixiert. Die herausgeführten Monofilamente stammen dabei von Doppelfäden, die der Reihe nach wechseln. Auf diese Weise wird ein besonders geschmeidiger Faden erhalten.

### Figurenbeschreibung

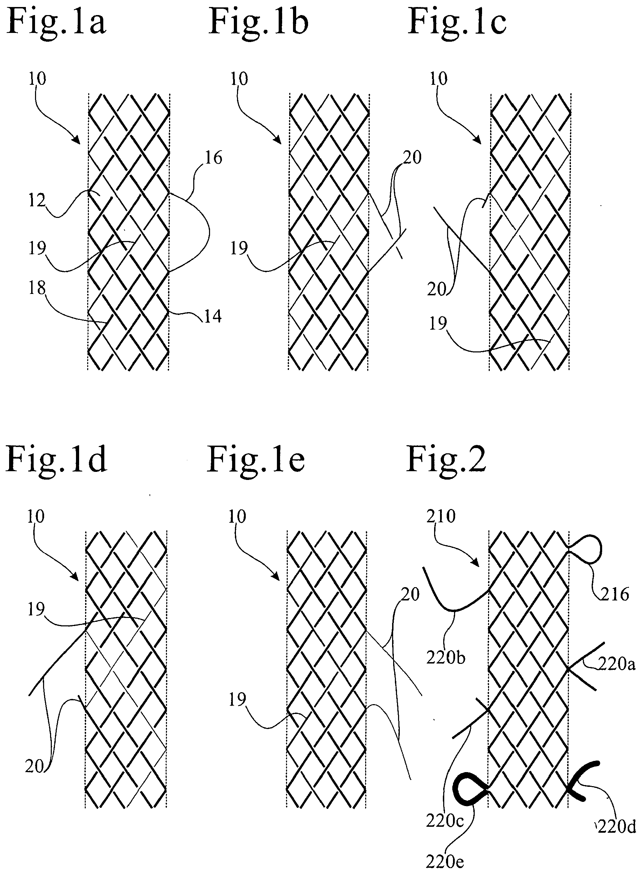

Figur 1 zeigt schematisch einen Ausschnitt aus einem Nahtmaterial **10** in Form eines Fadengeflechts **12** sowie verschiedene Formen von Fadengebilden **16**; **20**, die von der Oberfläche **14** des Fadengeflechts **12** abstehen. Das Fadengeflecht **12** kann ein Rund- oder Flachgeflecht sein. Das Fadengeflecht **12** ist aus Doppelfäden **18** (fett hervorgehoben) geflochten. Bei dem als Fadenschlinge ausgebildeten Fadengebilde **16** kann es sich um eine Flottung, Überfütterung oder Veloursschlinge handeln (Figur 1a). Die Fadenschlinge **16** kann durch Herausführen eines Einzelfadens **19** aus dem Fadengeflecht **12** und anschließendes Wiederhineinführen des Einzelfadens **19** in das Fadengeflecht **12** erzeugt werden. Dabei kann die Schlinge **16** einen Flechtfaden **18** (Figur 1a) oder gegebenenfalls mehrere Flechtfäden **18** "überspringen, ehe sie wieder in das Fadengeflecht **12** inkorporiert wird. Gegebenenfalls kann die Schlinge **16** auch keinen Flechtfaden **18** "überspringen", das heißt, ein herausgeführter Einzelfaden **19** wird unmittelbar wieder in das Fadengeflecht **12** eingefügt. Die Schlinge **16** dient mit besonderem Vorteil als Verankerungsstruktur zur Verankerung des Nahtmaterials **10** in einem biologischen Gewebe.

Im Rahmen der vorliegenden Erfindung kann es aber auch vorgesehen sein, dass die Schlinge **16** geöffnet wird, wodurch aus dem Fadengeflecht **12** herausragende Fadengebilde in Form von Fadenenden **20** erzeugt werden (Figuren 1b bis 1e). Bei Zug in die entgegengesetzte Richtung stellen sich die Fadenenden **20** auf und verankern auf diese Weise das Nahtmaterial **10** im Gewebe. Das Öffnen der Schlingen **16** kann prinzipiell durch verschiedene Möglichkeiten vorgenommen werden. Die Schlingen **16** können beispielsweise etwa mittig geöffnet werden. Dadurch entstehen zwei aus dem Fadengeflecht **12** herausragende Fadenenden **20**, welche in der Regel jeweils in sich entgegengesetzte Richtungen zeigen (Figur 1b). Normalerweise wird eines der beiden Fadenenden **20** möglichst dicht über dem Fadengeflecht **12** abgeschnitten oder gekappt. Die abgeschnittenen bzw. gekappten Fadenenden werden gegebenenfalls fixiert, damit sie sich nicht aus dem Geflecht lösen. Die Richtung der herausragenden Fadenenden **20** wird insbesondere durch den Flechtwinkel und die Länge der Fadenenden **20** bestimmt. Dies eröffnet die Möglichkeit, in Längsrichtung des Fadengeflechts **12** aus dem Fadengeflecht **12** herausragende Fadenenden **20** mit entgegengesetzten Richtungen auszubilden (Figuren 1c und 1d). Es ist aber auch möglich, beide durch das Öffnen jeweils einer Schlinge **16** erzeugten Fadenenden **20** als Verankerungsstrukturen des Nahtmaterials **10** stehenzulassen (Figur 1e). Die beiden Fadenenden **20,** die jeweils durch das Öffnen einer Schlinge **16** gebildet werden, können gegebenenfalls auch von unterschiedlicher Länge sein. Dies hängt davon ab, ob die Schlinge **16** mittig oder an einer davon abweichenden Position geöffnet wird. Die in den Figuren 1a-e beschriebenen Ausführungsformen hinsichtlich der Fadengebilde können sinngemäß auch auf ein Fadengeflecht aus Einzelfäden oder auf ein Fadengeflecht aus Einzel- und/oder Mehrfachfäden, insbesondere Doppel- und/oder Tripelfäden, übertragen werden.

Figur 2 zeigt schematisch weitere Variationsmöglichkeiten für aus einer Flechtstruktur **210** herausragende Fadengebilde **216; 220a-e** eines erfindungsgemäßen Nahtmaterials **210.** Die aus der Flechtstruktur **210** herausragenden oder abstehenden Fadengebilde **216; 220a-e** können nach Herstellung des Fadengeflechts in einem zusätzlichen Schritt versteift werden. Die Versteifung kann beispielsweise durch Verschweißen oder durch Beschichtung mit einem Polymer vorgenommen werden (verschweißte oder beschichtete Fadenenden **220d** und verschweißte oder beschichtete Schlingen **220e**).

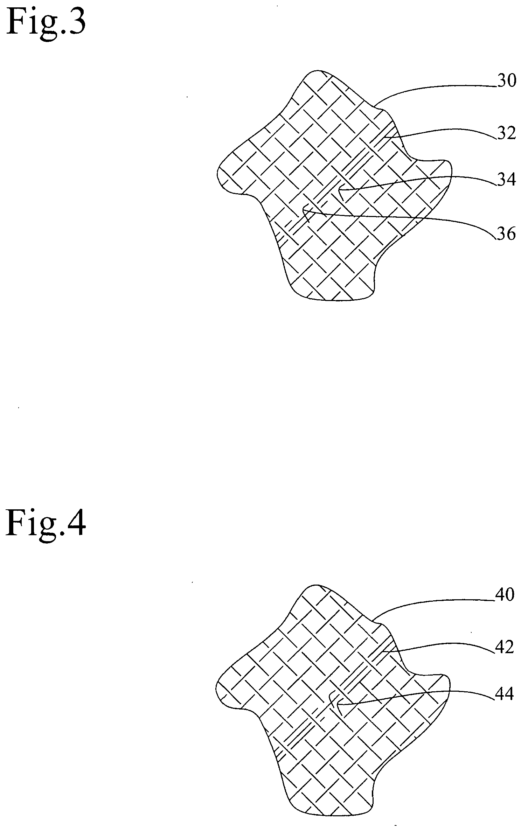

Figur 3 zeigt schematisch eine Draufsicht auf ein erfindungsgemäßes Nahtmaterial **30** mit Tripelfäden **32** (Scharfäden mit jeweils drei Fäden). Zwei Einzelfäden des Tripelfadens **32** sind an verschiedenen Stellen aus dem Geflecht herausgeführt und sind als abstehende Fadenenden **34** und **36** ausgebildet.

Figur 4 zeigt schematisch eine Draufsicht auf ein erfindungsgemäßes Nahtmaterial **40** mit Tripelfäden **42,** wobei zwei Einzelfäden des Tripelfadens **42** an derselben Stelle aus der Flechtstruktur herausgeführt sind. Die herausgeführten Einzelfäden sind ebenfalls als abstehende Fadenenden **44** ausgebildet.

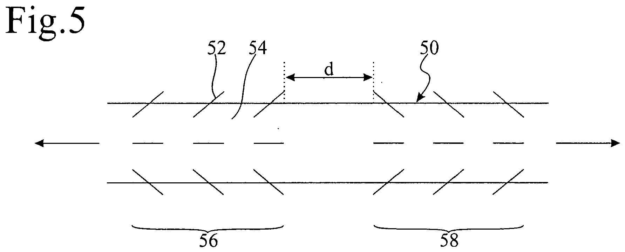

Figur 5 zeigt schematisch eine Seitenansicht eines erfindungsgemäßen Nahtmaterials **50,** dessen von der Flechtstruktur abstehende Fadengebilde **52** in Form von Fadenenden ausgebildet sind. Die Fadenenden **52** sind für einen ersten Flächen- bzw. Umfangsabschnitt **56** in Richtung Mitte der Flechtstruktur und für einen übrigen zweiten Flächen- bzw. Umfangsabschnitt **58** ebenso in Richtung Mitte der Flechtstruktur orientiert. Die Flächen- bzw. Umfangsabschnitte **56** und **58** können dabei einen bestimmten Abstand d zueinander aufweisen. Die Enden des Nahtmaterials **50** können zudem jeweils mit einer chirurgischen Nadel verbunden sein.

Das erfindungsgemäße Nahtmaterial kann gleichzeitig verschiedene Typen von abstehenden Fadengebilden aufweisen. Beispielsweise kann das Nahtmaterial sowohl Fadenschlingen als auch Fadenenden aufweisen, die aus der Flechtstruktur herausragen. Die Fadengebilde können zudem in unterschiedlichen Längen und Größenverhältnissen vorliegen.

## Patentansprüche

1. Chirurgisches multifiles Nahtmaterial (10; 210; 30; 40; 50) in Form eines Fadengeflechts, wobei das Nahtmaterial zur Verankerung in biologischen Geweben aus dem Fadengeflecht herausragende Fadengebilde (16; 20; 216; 220a-e; 34; 36; 44) aufweist, wobei es sich bei den herausragenden Fadengebilden (16; 20; 216; 220a-e; 34; 36; 44) um Fadenschlingen (16; 216; 220e) oder um geöffnete Fadenschlingen (20; 220a-d; 34; 36; 44) handelt, **dadurch gekennzeichnet, dass** es sich bei den herausragenden Fadengebilden um Einzelfäden, die von Mehrfachfäden stammen, handelt.

2. Chirurgisches multifiles Nahtmaterial (10; 210; 30; 40; 50) nach Anspruch 1, **dadurch gekennzeichnet, dass** es sich bei den Fadenschlingen um Flottungen, Überfütterungen und/oder Veloursschlingen handelt.

3. Chirurgisches multifiles Nahtmaterial (10; 210; 30; 40; 50) nach Anspruch 1 oder 2, **dadurch gekennzeichnet, dass** es sich bei den geöffneten Fadenschlingen um geöffnete Flottungen, geöffnete Überfüttungen und/oder geöffnete Veloursschlingen handelt.

4. Chirurgisches multifiles Nahtmaterial (10; 210; 30; 40; 50) nach einem der vorhergehenden Ansprüche, **dadurch gekennzeichnet, dass** das Fadengeflecht aus mono- und/oder multifilen Fäden gebildet ist.

5. Chirurgisches multifiles Nahtmaterial (10; 210; 30; 40; 50) nach einem der vorhergehenden Ansprüche, **dadurch gekennzeichnet, dass** das Fadengeflecht Mehrfachfäden, insbesondere Doppelfäden (18) und/oder Tripelfäden (32; 42), aufweist.

6. Chirurgisches multifiles Nahtmaterial (10; 210; 30; 40; 50) nach einem der vorhergehenden Ansprüche, **dadurch gekennzeichnet, dass** es sich bei den herausragenden Fadengebilden um Einzelfäden, die von Doppel- und/oder Tripelfäden stammen, handelt.

7. Chirurgisches multifiles Nahtmaterial (10; 210; 30; 40; 50) nach einem der vorhergehenden Ansprüche, **dadurch gekennzeichnet, dass** die Fäden, deren Gebilde (16; 20; 216; 220a-e; 34; 36; 44) aus dem Fadengeflecht herausragen, eine höhere Biegesteifigkeit aufweisen als die restlichen Fäden des Fadengeflechts, wobei die Fäden, deren Gebilde (16; 20; 216; 220a-e; 34; 36; 44) aus dem Fadengeflecht herausragen, vorzugsweise einen höheren Titer als die restlichen Fäden des Fadengeflechts aufweisen.

8. Chirurgisches multifiles Nahtmaterial (10; 210; 30; 40; 50) nach einem der vorhergehenden Ansprüche, **dadurch gekennzeichnet, dass** das Fadengeflecht aus einer ungeraden Anzahl von Fäden gebildet und ein Flachgeflecht oder aus einer geraden Anzahl von Fäden gebildet und ein Rund- oder Schlauchgeflecht ist.

9. Chirurgisches multifiles Nahtmaterial (10; 210; 30; 40; 50) nach Anspruch 8, **dadurch gekennzeichnet, dass** das Rund- oder Schlauchgeflecht eine Seele aufweist.

10. Verfahren zur Herstellung eines Nahtmaterials (10; 210; 30; 40; 50) nach einem der vorhergehenden Ansprüche, wobei zur Herstellung des Fadengeflechts Fäden unter Ausbildung einer Flechtstruktur miteinander geflochten werden und einzelne Fäden zur Ausbildung von aus der Flechtstruktur herausragenden Fadengebilden (16; 20; 216; 220a-e; 34; 36; 44) während des Flechtens aus der sich bildenden Flechtstruktur herausgeführt werden, wobei die herausgeführten Fäden unter Ausbildung von Schlingen (16; 216; 220e) wieder in die Flechtstruktur hineingeführt und gegebenenfalls die Schlingen (16; 216; 220e) geöffnet werden.

11. Verfahren nach Anspruch 10, **dadurch gekennzeichnet, dass** andere Fäden in die Flechtstruktur eingeführt werden, um die Fadenzahl im Wesentlichen konstant zu halten.

12. Verfahren nach Anspruch 10 oder 11, **dadurch gekennzeichnet, dass** die Fäden beim Flechten mindestens doppelt genommen werden.

13. Chirurgisches Kit, umfassend ein Nahtmaterial (10; 210; 30; 40; 50) nach einem der Ansprüche 1 bis 9 und zumindest eine chirurgische Nadel.

## Claims

1. Surgical multifilament suture material (10; 210; 30; 40; 50) in the form of a filamentary braid, wherein the suture material includes filamentary elements (16; 20; 216; 220a-e; 34; 36; 44) projecting from the filamentary braid for anchoring in biological tissues, wherein the projecting filamentary elements (16; 20; 216; 220a-e; 34; 36; 44) are filamentary loops (16; 216; 220e) or opened filamentary loops (20; 220a-d; 34; 36; 44), **characterized in that** the projecting filamentary elements are single filaments originating from multiplied filaments.

2. Surgical multifilament suture material (10; 210; 30; 40; 50) according to claim 1, **characterized in that** the filamentary loops comprise floats, overfeeds and/or velour loops.

3. Surgical multifilament suture material (10; 210; 30; 40; 50) according to claim 1 or 2, **characterized in that** the opened filamentary loops comprise opened floats, opened overfeeds and/or opened velour loops.

4. Surgical multifilament suture material (10; 210; 30; 40; 50) according to any of the preceding claims, **characterized in that** the filamentary braid is formed from monofilaments and/or multifilaments.

5. Surgical multifilament suture material (10; 210; 30; 40; 50) according to any of the preceding claims, **characterized in that** the filamentary braid includes multiplied filaments, more particularly doubled filaments (18) and/or tripled filaments (32; 42).

6. Surgical multifilament suture material (10; 210; 30; 40; 50) according to any of the preceding claims, **characterized in that** the projecting filamentary elements comprise single filaments originating from doubled and/or tripled filaments.

7. Surgical multifilament suture material (10; 210; 30; 40; 50) according to any of the preceding claims, **characterized in that** the filaments whose elements (16; 20; 216; 220a-e; 34; 36; 44) project from the filamentary braid have a higher flexural stiffness than the remaining filaments of the filamentary braid, wherein the filaments whose elements (16; 20; 216; 220a-e; 34; 36; 44) project from the filamentary braid preferably have a higher linear density than the remaining filaments of the filamentary braid.

8. Surgical multifilament suture material (10; 210; 30; 40; 50) according to any of the preceding claims, **characterized in that** the filamentary braid is composed of an odd number of filaments and is a flat braid, or is composed of an even number of filaments and is a round or tubular braid.

9. Surgical multifilament suture material (10; 210; 30; 40; 50) according to claim 8, **characterized in that** the round or tubular braid includes a core.

10. Method for producing a suture material (10; 210; 30; 40; 50) according to any of the preceding claims, wherein for producing the filamentary braid filaments are braidingly intertwined to form a braided structure and individual filaments are led out of the braided structure, which forms during the braiding, for forming of filamentary elements (16; 20; 216; 220a-e; 34; 36; 44) projecting from the braided structure, wherein the filaments, which have been led out, are led back into the braided structure with the formation of loops (16; 216; 220e) and, optionally, the loops (16; 216; 220e) are opened.

11. Method according to claim 10, **characterized in that** other filaments are led into the braided structure in order to keep the number of filaments substantially constant.

12. Method according to claim 10 or 11, **characterized in that** the filaments are taken at least doubled in the braiding operation.

13. Surgical kit, comprising a suture material (10; 210; 30; 40; 50) according to any one of claims 1 to 9 and at least a surgical needle.

## Revendications

1. Matériau de suture chirurgical à fils multiples (10; 210; 30; 40; 50) sous la forme d'une tresse de fils, dans lequel le matériau de suture présente pour l'ancrage dans des tissus biologiques des pièces de fils (16; 20; 216; 220a-e; 34; 36; 44) sortant de la tresse de fils, dans lequel les pièces de fils sortantes (16; 20; 216; 220a-e; 34; 36; 44) sont des boucles de fils (16; 216; 220e) ou des boucles de fils ouvertes (20; 220a-d; 34; 36; 44), **caractérisé en ce que** les pièces de fils sortantes sont des fils individuels, qui proviennent de fils multiples.

2. Matériau de suture chirurgical à fils multiples (10; 210; 30; 40; 50) selon la revendication 1, **caractérisé en ce que** les boucles de fils sont des flottages, des doublures et/ou des boucles velours.

3. Matériau de suture chirurgical à fils multiples (10; 210; 30; 40; 50) selon la revendication 1 ou 2, **caractérisé en ce que** les boucles de fils ouvertes sont des flottages ouverts, des doublures ouvertes et/ou des boucles velours ouvertes.

4. Matériau de suture chirurgical à fils multiples (10; 210; 30; 40; 50) selon l'une quelconque des revendications précédentes, **caractérisé en ce que** la tresse de fils est formée de fils mono- et/ou multibrins.

5. Matériau de suture chirurgical à fils multiples (10; 210; 30; 40; 50) selon l'une quelconque des revendications précédentes, **caractérisé en ce que** la tresse de fils présente des fils multiples, en particulier des fils doubles (18) et/ou des fils triples (32; 42) .

6. Matériau de suture chirurgical à fils multiples (10; 210; 30; 40; 50) selon l'une quelconque des revendications précédentes, **caractérisé en ce que** les pièces de fils sortantes sont des fils individuels, qui proviennent de fils doubles et/ou de fils triples.

7. Matériau de suture chirurgical à fils multiples (10; 210; 30; 40; 50) selon l'une quelconque des revendications précédentes, **caractérisé en ce que** les fils, dont les pièces (16; 20; 216; 220a-e; 34; 36; 44) sortent de la tresse de fils, présentent une rigidité à la flexion plus élevée que le reste des fils de la tresse de fils, dans lequel les fils, dont les pièces (16; 20; 216; 220a-e; 34; 36; 44) sortent de la tresse de fils, présentent de préférence un titre plus élevé que le reste des fils de la tresse de fils.

8. Matériau de suture chirurgical à fils multiples (10; 210; 30; 40; 50) selon l'une quelconque des revendications précédentes, **caractérisé en ce que** la tresse de fils est formée d'un nombre impair de fils et est une tresse plate ou est formée d'un nombre pair de fils et est une tresse ronde ou creuse souple.

9. Matériau de suture chirurgical à fils multiples (10; 210; 30; 40; 50) selon l'une quelconque des revendications précédentes, **caractérisé en ce que** la tresse ronde ou creuse souple présente une âme.

10. Procédé de fabrication d'un matériau de suture (10; 210; 30; 40; 50) selon l'une quelconque des revendications précédentes, dans lequel pour la fabrication de la tresse de fils on tresse des fils les uns avec les autres en formant une structure de tresse et on fait sortir quelques fils pendant le tressage hors de la structure de tresse en formation pour former des pièces de fils (16; 20; 216; 220a-e; 34; 36; 44) sortant de la structure de tresse, dans lequel les fils sortants sont de nouveau rentrés dans la structure de tresse pour former des boucles (16; 216; 220e) et on ouvre éventuellement les boucles (16; 216; 220e).

11. Procédé selon la revendication 10, **caractérisé en ce que** l'on introduit d'autres fils dans la structure de tresse, afin de maintenir le nombre de fils essentiellement constant.

12. Procédé selon la revendication 10 ou 11, **caractérisé en ce que** l'on prend les fils au moins en double lors du tressage.

13. Kit chirurgical, comprenant un matériau de suture (10; 210; 30; 40; 50) selon l'une quelconque des revendications 1 à 9 et au moins une aiguille chirurgicale.
